# EUROPÄISCHE PATENTSCHRIFT

(11) **EP 0 536 710 B1**
(45) Veröffentlichungstag und Bekanntmachung des Hinweises auf die Patenterteilung: **08.05.1996**
(21) Anmeldenummer: 92117092.4
(22) Anmeldetag: 07.10.1992
(51) Int. Cl.: C07C 237/06, C07C 237/22, C07C 271/22, C07K 1/04, C07K 1/06

(54) **Aminosäurederivate zur Peptidsynthese**
Amino acid derivatives for peptide synthesis
Dérivés d'acides aminés pour la synthèse peptidique

(30) Priorität: 11.10.1991 CH 911/91
(43) Veröffentlichungstag der Anmeldung: 14.04.1993
(73) Patentinhaber: BACHEM FEINCHEMIKALIEN AG, CH-4416 Bubendorf (CH)
(72) Erfinder: Gosteli, Jacques, CH-4059 Basel (CH); Sax, Beat, CH-4416 Bubendorf (CH); Dick, Fritz, CH-4416 Bubendorf (CH); Tanner, Rudolf, CH-4416 Bubendorf (CH)

(56) Entgegenhaltungen:
- EP-A- 0 394 194
- CHEMICAL ABSTRACTS, vol. 114, no. 19, 13. Mai 1991, Columbus, Ohio, US;abstract no. 186049y, SIEBER, PETER ET AL. 'Protection of carboxamide functions by the trityl residue. Application to peptide synthesis.' Seite 848 ;Spalte 1 ; & TETRAHEDRON LETTERS Bd. 32, Nr. 6, Juni 1991, Seiten 739-742
- TETRAHEDRON LETTERS Bd. 28, Nr. 48, 1987, Seiten 6031 - 6034 PETER SIEBER ETAL. 'protection of histidine in peptide synthesis: a reassessment of the trityl group.'

## Beschreibung

Die Erfindung betrifft N-Methyltrityl-Carbamoylgruppen enthaltende Aminosäurederivate, ihre höheren Homologen und ihre Anwendung in der Peptidsynthese.

Seit langem ist bekannt, dass die Amidgruppen der Aminosäuren Asparagin und Glutamin bei der Peptidsynthese häufig zu unerwünschten Nebenreaktionen führen (Gish et al. 1956, Ressler 1956, Katsoyannis et al. 1958, etc.).

Schutz der Amidfunktion verhindert diese Nebenreaktionen. Verwendung gefunden haben bisher die 4,4'-Dimethoxydiphenylmethyl (Mbs), die 2,4,6-Trimethoxybenzyl (Tmob) und seit kurzem auch die Triphenylmethyl (Trityl) -Schutzgruppe, welche letztere Gruppe, im Gegensatz zu den übrigen, bei der Abspaltung nicht zu Nebenprodukten führt. (Lit.: P. Sieber + B. Riniker, Symposium on Solid Phase Synthesis, Oxford,U.K.; Sept. 1989).

Erfahrungen mit der Tritylschutzgruppe liessen es als wünschenswert erscheinen, eine solche Gruppe zu finden, die die Vorteile der Tritylgruppe beinhaltet, aber schneller als diese abgespaltet werden kann. Darin besteht die Aufgabe der vorliegenden Erfindung.

Sie betrifft Verbindungen der Formel I, 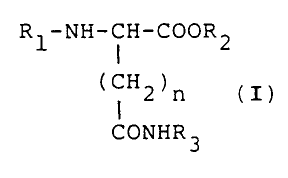
worin n gleich 1 oder 2 ist, R₁ Wasserstoff oder eine Aminoschutzgruppe, R₂ Wasserstoff oder eine Carboxylschutzgruppe und R₃ 4-Methyltriphenylmethyl, 4,4'-Dimethyltriphenylmethyl und 4,4',4''-Trimethyltriphenylmethyl bedeuten, Salze von solchen Verbindungen und reaktionsfähige Carbonsäurederivate von solchen Verbindungen der Formel I, worin R₂ für Wasserstoff steht. Die Konfiguration am chiralen HC-Atom ist beliebig, D, L oder D,L.

Verbindungen der Formel I führen bei der Spaltung nicht zur Alkylierung von Tryptophan, falls dieses im Peptid zugegen ist. Sie sind leicht herstellbar und ebenso leicht mit Trifluoressigsäure oder diese Säure enthaltenden Gemischen abspaltbar. In der Reaktionsgeschwindigkeit der Spaltung übertreffen die Verbindungen der Formel I die analogen, tritylierten Verbindungen um das zwei- bis mehrfache (Fünffache). Sie sind wie die Tritylverbindungen besonders geeignet für die Festphasen-Peptidsynthesemethode, bei der basenlabile Schutzgruppen R₁ zum Schutz der α-Aminogruppen verwendet werden, wie z.B. die 9-Fluorenmethyloxycarbonyl (Fmoc)-Gruppe. Selbstverständlich kommen als Aminoschutzgruppen R₁ aber auch alle in der Peptidsynthese üblichen Gruppen wie Acylreste von Kohlensäurehalbestern, insbesondere tert.-Butyloxycarbonyl, Benzyloxycarbonyl (gegebenenfalls substituiert), 2-Halogenniederalkoxycarbonyl oder Allyloxycarbonyl in Frage.

Carboxylschutzgruppen R₂ sind solche, die in der Peptidchemie üblicherweise zur Anwendung kommen, wie z.B. Niederalkyl wie Methyl oder Ethyl, tert.alkyl, wie tert.butyl, benzyl, nitrobenzyl, methoxybenzyl, benzhydryl, allyl, cycloalkyl, phenyl, βββ-trihalogenethyl, β-triniederalkylsilylethyl, wie β-trimethylsilylethyl.

Die Gruppe R₂ kann aber auch die Funktion der Aktivierung der Carboxylgruppe ausüben. Derartige Derivate der Formel I können dann zur Acylierung, wie z.B. Knüpfung einer Peptidbindung verwendet werden. Man spricht dann von aktivierten Estern, wenn R₂ ein ungesättigter Rest ist, dessen Doppelbindung nächstmöglich zur Carbonylgruppe situiert ist, was z.B. bei Vinylestern, Phenylestern, substituierten Phenylestern, wie z.B. Pentafluorphenylestern der Fall ist. Ebenso geeignet sind andere aktivierte Ester, wie z.B. Cyanomethylester, Thioester, wie z.B. Thiophenylester, Hydroxysuccinimidester, N-Hydroxyphthalimidester, 1-Hydroxybenztriazolester, 3-Hydroxy-3,4-dihydro-4-oxo-1,2,3-benzotriazinester. R₂ kann auch so gestaltet sein, dass die Funktion -COOR₂ in Formel I als Anhydrid aufgefasst werden kann. In Frage kommen gemischte Anhydride, z.B. solche, die durch Umsetzung mit Kohlensäurehalbesterchloriden erhalten werden, ferner symmetrische Anhydride, Anhydride mit anorganischen Säuren, wie z.B. Säureazide, Azolide, Iminoanhydride, wie sie z.B. durch Anlagerung von Carbodiimiden an Verbindung der Formel I (R₂ = H) erhalten werden. Diese Anhydride dienen demselben Zweck wie die aktivierten Ester, nämlich der Acylierung.

Die hier verwendeten Abkürzungen für die Aminosäuren sind die allgemein üblichen. So bedeuten Asp Asparaginsäure, Asn Asparagin, Gln Glutamin, Glu Glutaminsäure, Ser Serin, Leu Leucin, Gly Glycin, Lys Lysin, His Histidin, Pro Prolin, Tyr Tyrosin, Trp Tryptophan, Arg Arginin, Val Valin und Ile Isoleucin.

Gemäss den international anerkannten Nomenklaturregeln bezeichnen die Abkürzungen für die Aminosäuren, z.B. die obengenannten Abkürzungen in diesem Text die freie Säure, und, falls nicht anders angegeben, in der L-Konfiguration. Die α-Aminogruppe ist an der linken Seite der Abkürzung, die Carboxygruppe an der rechten Seite zu denken. Substituenten (Schutzgruppen) in der Seitenkette von Aminosäuren werden unmittelbar hinter das Aminosäurekürzel in Klammern gesetzt. Somit steht z.B. Z-Asn(MeTrt)-OH für die Verbindung der Formel Ia. 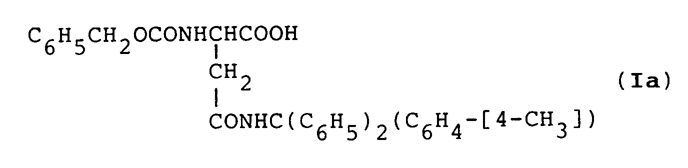
Z = Cbz sind übliche Abkürzungen für den Carbobenzoxyrest
MeTrt wird vorgeschlagen als Abkürzung für die 4-Methyltrityl-Schutzgruppe (den 4-Methyltritylrest)
Die durch Verwendung der Verbindungen der Formel I bei der Peptidsynthese erzielten Vorteile sind im experimentellen Teil illustriert. Sie beruhen auf der rascheren Abspaltung der MeTrt-Gruppe im Vergleich zur Trt-Gruppe.

Die Verbindungen der Formel I können als Bausteine bei der Synthese von Peptiden, die Glutamin- und/oder Asparaginreste enthalten, oder als Zwischenprodukte zur Herstellung dieser Bausteine verwendet werden. Zur Peptidsynthese an fester Phase verwendet man diejenigen Verbindungen der Formel I, worin R₁ eine Aminoschutzgruppe, wie insbesondere 9-Fluorenylmethyloxycarbonyl oder tert.Butoxycarbonyl, R₂ Wasserstoff und R₃ 4-Methyltrityl bedeuten, und reaktionsfähige Carbonsäurederivate davon.

Die erfindungsgemässe Amidschutzgruppe R₃ ist stabil bei der katalytischen Hydrierung unter Bedingungen, unter denen Benzyloxycarbonyl (als R₁) oder Benzyl als Carboxylschutzgruppe (als R₂) abgespalten werden. Die Schutzgruppe R₃ kann z.B. mit Trifluoressigsäure-Dichlormethan (1:1) im Temperaturbereich zwischen -20°C und +50°C, z.B. bei +30°C, abgespalten werden.

Die Verbindungen der Formel I werden in an sich bekannter Weise hergestellt. Das Herstellungsverfahren ist dadurch gekennzeichnet, dass man eine Verbindung der Formel II, 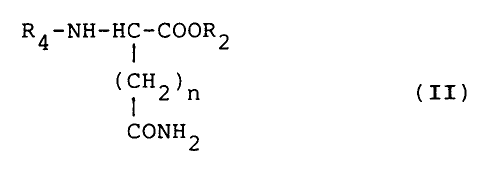
worin R₄ Wasserstoff oder eine unter den Reaktionsbedingungen stabile Aminoschutzgruppe bedeutet und n und R₂ für die obengenannten Bedeutungen stehen, mit einer Verbindung der Formel III,

R₃OH (III)

worin R₃ die oben genannte Bedeutung hat, umsetzt und aus dem erhaltenen Produkt allenfalls diejenigen Schutzgruppen abspaltet, die nicht Bestandteil des gewünschten Endprodukts der Formel I sind.

Das Verfahren wird im folgenden näher erläutert:
In einem geeigneten organischen Lösungsmittel, wie z.B. Essigsäure, wird die Reaktion in Gegenwart katalytischer Mengen einer starken Säure, vorzugsweise Schwefelsäure, sowie in Gegenwart eines wasserentziehenden Mittels, wie z.B. Essigsäureanhydrid, im Temperaturbereich zwischen 0°C und 100°C, bevorzugt zwischen 20°C und 70°C, z.B. bei 50°C durchgeführt.

Eine Aminoschutzgruppe R₄ ist eine unter sauren Bedingungen stabile Aminoschutzgruppe, wie z.B. Trifluoracetyl oder vorzugsweise Benzyloxycarbonyl oder 9-Fluorenylmethyloxycarbonyl.

Wenn im gewünschten Endprodukt der Formel I R₁ und/oder R₂ für Wasserstoff stehen, so müssen die Schutzgruppen R₄ und R₂ abgespalten werden.
Carboxylschutzgruppen R₂, die unter den Reaktionsbedingungen stabil sind, können, falls erwünscht und so es sich um einfache Gruppen wie Methyl, Ethyl oder Allyl handelt, durch Verseifung abgespalten werden.

Trihalogenacetyl, wie z.B. Trifluoracetyl als Aminoschutzgruppe R₄ wird durch milde Verseifung, wie z.B. mit Sodalösung abgespalten.
Benzyloxycarbonyl als Aminoschutzgruppe R₄ wird durch Hydrogenolyse in Gegenwart eines Schwermetallkatalysators, wie z.B. Palladium auf Bariumsulfat abgespalten.

9-Fluorenylmethyloxycarbonyl als Aminoschutzgruppe R₄ wird mittels einer Lösung eines Amins, z.B. Piperidin in Dimethylacetamid abgespalten.

Die Ausbeuten über drei Stufen betragen etwa 45 % der Theorie.

Verbindungen der Formel I können auch, insbesondere wenn R₁ und/oder R₂ gleich Wasserstoff ist, als Salze vorliegen, bzw. durch Zugabe von Säuren bzw. Basen, in Salze übergeführt werden, wobei vorzugsweise stöchiometrische Mengen dieser Agenzien eingesetzt werden, um die Salzbildung zu vervollständigen. Innere Salze der Formel I (R₁ = R₂ = Wasserstoff) können aus anderen Salzen durch Neutralisation ihrer Lösung bis zum isoelektrischen Punkt und Isolierung hergestellt werden.

Die Erfindung betrifft auch die Verwendung der Verbindungen der Formel I, insbesondere die Verwendung derjenigen Verbindungen der Formel I, worin R₁ eine Aminoschutzgruppe, wie Benzyloxycarbonyl, tert.Butoxycarbonyl oder insbesondere 9-Fluorenmethyloxycarbonyl, R₂ Wasserstoff und R₃ 4-Methyltrityl bedeuten, sowie die Verwendung reaktionsfähiger Carbonsäurederivate dieser Verbindungen zur Synthese von Peptiden, insbesondere an fester Phase.

Die Erfindung betrifft auch Stoffe, die einen oder mehrere bivalente Reste der Formel IV, 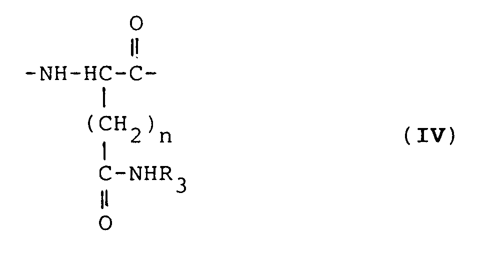
worin n für 1 oder 2 steht und R₃ 4-Methyltrityl bedeutet, enthalten. Bei diesen Verbindungen handelt es sich um Peptidderivate, bzw. -fragmente, wie sie bei der Peptidsynthese als Zwischenprodukte anfallen; sie können auch an ein Syntheseharz gebunden sein.

Die nachfolgenden Beispiele illustrieren die Erfindung, ohne sie in irgend einer Weise einzuschränken.

### Verwendete Abkürzungen

| | |
|---|---|
| Fmoc OSu | 9-Fluorenylmethyloxycarbonyl-N-Hydroxysuccinimidester |
| Z (Cbz) | Benzyloxycarbonyl |
| Me Trt *Mtt | 4-Methyltrityl |
| Fmoc | 9-Fluorenylmethyloxycarbonyl |
| Asn | Asparagin |
| Gln | Glutamin |

| | |
|---|---|
| *Diese Abkürzung wurde neu vorgeschlagen für 4-Methyltrityl sh. M. Friede et al., Peptide Research 5, 145 (1992). | |

### Beispiel 1: Z-Asn (MeTrt)OH

Ein Gemisch von 66,5 g Z-Asn-OH (250 mM), 137,2 g 4-Methyltriphenylcarbinol (500 mM), 750 ml Essigsäure und 47 ml (500 mM) Acetanhydrid wird bei Raumtemperatur 5 Minuten gerührt. Nach Zugabe von 1,25 ml konz. Schwefelsäure wird auf 50° C erhitzt; es entsteht nach 15 Minuten eine klare, gelbe Lösung. Nach 2½ h wird abgekühlt und in 5 l Eiswasser ausgegossen. Der Niederschlag wird abfiltriert, gut mit Wasser gewaschen und in etwa 500 ml Essigsäureethylester gelöst. Die organische Phase wird mit einer gesättigten wässrigen Natriumchloridlösung gewaschen, über Natriumsulfat getrocknet und unter vermindertem Druck auf 300 ml Oel eingeengt. Z-Asn(MeTrt)OH wird durch Zugabe von Diisopropylether zum Kristallisieren gebracht.
Smp. 187° - 197° C.
Ausbeute: 75 %.

### Beispiel 2: H-Asn(MeTrt)OH

Eine Suspension von 52,3 g Z-Asn(MeTrt)OH (100 mM) in 400 ml Methanol und 100 ml ln wässriger Salzsäure wird in Gegenwart von 2 g eines 10 %-igen Palladium-auf-Kohle-Katalysators bei Raumtemperatur und Normaldruck hydriert. Nach beendeter Wasserstoffaufnahme wird der Katalysator abfiltriert, mit Methanol ausgewaschen und das Filtrat nach Zusatz von 13,9 ml Triethylamin (100 mM) und 100 ml Wasser auf ca. 300 ml eingeengt; der kristalline Niederschlag wird abfiltriert und mit Wasser chloridfrei gewaschen. Das erhaltene H-Asn(MeTrt)OH enthält 0,5 Mol bis 1 Mol Wasser und zersetzt sich ab 222 ° C.
Ausbeute: 80 %.

### Beispiel 3: Fmoc-Asn(MeTrt)OH

19,9 g H-Asn(MeTrt)OH x 0,5 H₂O (50 mM) werden in einer Lösung von 10,6 g Natriumcarbonat (100 mM) in 125 ml H₂O und 25 ml Dioxan suspendiert. Zu dieser Suspension wird bei Raumtemperatur unter Rühren innert 1 h eine Lösung von 16,9 g Fmoc OSu (50 mM) in 68⁻ ml Dioxan zugetropft. Nach beendeter Reaktion (über Nacht) wird das Reaktionsgemisch mit H₂O verdünnt und mehrfach mit Ether extrahiert; anschliessend wird die H₂O-Phase mit 400 ml Essigester überschichtet, mit halbkonzentrierter Salzsäure angesäuert (pH 2-3), die organische Phase mit halbgesättigter Kochsalzlösung gewaschen und über Natriumsulfat getrocknet. Nach diesem Einengen unter vermindertem Druck zum Oel (ca. 50 g), wird aus Ether und Diisopropylether kristallisiert.
Smp. ab 190° - 202° C (Zersetzung).
Ausbeute: 75 %.

### Beispiel 4: Z-Gln(MeTrt)OH

Ein Gemisch von 140,14 g Z-Gln-OH (0,5 Mol), 274,4 g 4-Methyltriphenylcarbinol (1 Mol), in 1,5 l Essigsäure und 93,4 ml Essigsäureanhydrid (1 mol) wird 7 Minuten bei Raumtemperatur gerührt. Nach Zugabe von 2,5 ml konz. Schwefelsäure wird das Reaktionsgemisch auf 50° C erhitzt; es entsteht nach 15 Minuten eine klare, gelbe Lösung. Nach 2½ h wird abgekühlt und in 5 l Eiswasser ausgegossen. Der Niederschlag wird abfiltriert, gut mit Wasser gewaschen und in etwa 1 l Essigsäureethylester gelöst. Die organische Phase wird mit einer gesättigten Natriumchloridlösung gewaschen, über Natriumsulfat getrocknet und unter vermindertem Druck auf 400 ml Oel eingeengt. Das Z-Gln(MeTrt)OH wird durch Zugabe von Diisopropylether zum Kristallisieren gebracht.
Smp. 187° - 197° C.
Ausbeute: 75 %.

### Beispiel 5: H-Gln(MeTrt)OH

Eine Susp. von 107,3 g Z-Gln(MeTrt)OH (200 mM) in 800 ml Methanol und 200 ml ln wässriger Salzsäure wird in Gegenwart von 0,4 g eines 10 %-igen Palladium-auf-Kohle-Katalysators bei Raumtemperatur und Normaldruck hydriert. Nach beendeter Wasserstoffaufnahme wird der Katalysator abfiltriert, mit Methanol ausgewaschen und das Filtrat nach Zusatz von 27,9 ml Triethylamin (200 mM) + 200 ml H₂O auf ca. 600 ml eingeengt; der kristalline Niederschlag wird abfiltriert und mit Wasser chloridfrei gewaschen. Das erhaltene H-Gln(MeTrt)OH enthält 0,5 Mol bis 1 Mol Wasser und zersetzt sich langsam ab 231° C.
Ausbeute: 85 %.

### Beispiel 6: Fmoc Gln(MeTret)OH

41,15 g H-Gln(MeTrt)OH x 0,5 H₂O (100 mM) wurden suspendiert in einer Lösung von 21,2 g Natriumcarbonat (200 mM) in 250 ml H₂O und 50 ml Dioxan.
Zu dieser Suspension wird bei Raumtemperatur unter Rühren innert einer Stunde eine Lösung von 33,7 g Fmoc OSu (100 mM) in 135 ml Dioxan zugetropft.
Nach beendeter Reaktion (über Nacht) wird das Reaktionsgemisch mit H₂O verdünnt und mehrfach mit einem Gemisch aus Diisopropylether/Diethylether (1:1) extrahiert. Anschliessend wird die Wasserphase mit 800 ml Essigester überschichtet, mit halbkonzentrierter Salzsäure auf pH 2-3 angesäuert, die organische Phase mit halbgesättigter Kochsalzlösung extrahiert und über Natriumsulfat getrocknet, unter vermindertem Druck zum Oel eingeengt und aus Ether kristallisiert.
Smp. ab 108° C (Zersetzung bis 144° C).
Ausbeute: 75 %.

## Patentansprüche

1. Verbindung der Formel I, 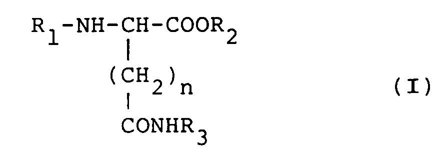 worin n gleich 1 oder 2 ist, R₁ Wasserstoff oder eine Aminoschutzgruppe, R₂ Wasserstoff, eine Carboxylschutzgruppe oder eine carboxylaktivierende Gruppe und R₃ 4-Methyltriphenylmethyl, 4,4'-Dimethyltriphenylmethyl, 4,4',4''-Trimethyltriphenylmethyl bedeuten und deren Salze.

2. Verbindung gemäß Anspruch 1 der Formel I, worin n, R₂ und R₃ die unter Anspruch 1 angegebene Bedeutung haben und R₁ den 9-Fluorenylmethoxycarbonylrest bedeutet.

3. Verbindung gemäß Anspruch 1 der Formel I, worin n, R₂ und R₃ die unter Anspruch 1 angegebene Bedeutung haben und R₁ den Carbobenzoxyrest bedeutet.

4. Verbindung gemäß Anspruch 1 der Formel I, worin n, R₂ und R₃ die unter Anspruch 1 angegebene Bedeutung haben und R₁ Wasserstoff bedeutet.

5. Verbindung der Formel V, 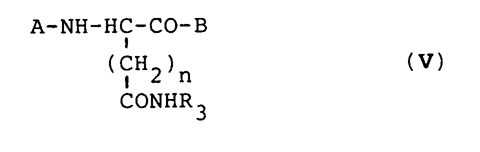 worin n und R₃ die unter Anspruch 1 angegebene Bedeutung haben und A, bzw. B, den aminoendständigen, bzw. carboxylendständigen Rest eines Peptids oder Peptidfragments (beides allenfalls polymergebunden) bedeuten.

## Claims

1. A compound according to formula I: 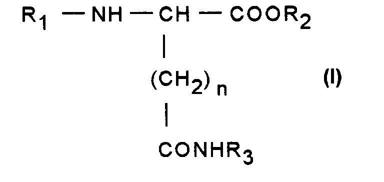 in which n is an integer selected from 1 and 2, R₁ is selected from hydrogen and amino protecting groups, R₂ is selected from hydrogen, carboxyl protecting groups and carboxyl activating groups, and R₃ is selected from 4-methyltriphenylmethyl, 4,4'-dimethyltriphenylmethyl, and 4,4',4''-trimethyltriphenylmethyl.

2. A compound according to claim 1 and of formula I, wherein n, R₂ and R₃ represent the subjects as indicated in claim 1 and R₁ stands for the 9-fluorenemethoxycarbonyl residue.

3. A compound according to claim 1 and of formula I, wherein n, R₂ and R₃ represent the subjects as indicated in claim 1 and R₁ stands for the carbobenzoxy residue.

4. A compound according to claim 1 and of formula I, wherein n, R₂ and R₃ represent the subjects as indicated in claim 1 and R₁ stands for hydrogen.

5. A compound of formula V: 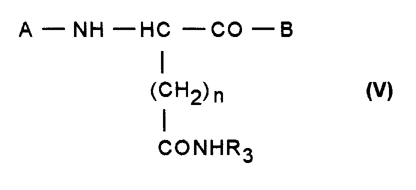 wherein n and R₃ represent the subjects defined in claim 1 and A and B stand for the residue of a peptide or peptide fragment (both optionally bound to a polymer) at the amino terminus or carboxyl end of a peptide, respectively.

## Revendications

1. Un composé selon formule I: 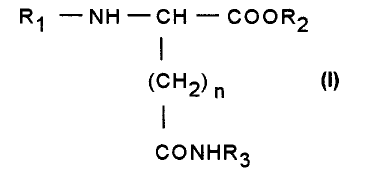 dans laquelle n représente le nombre 1 ou 2, R₁ représente hydrogène ou un groupe protecteur, R₂ représente hydrogène, un groupe bloquant la fonction carboxylique ou activant la fonction carboxylique, R₃ représente 4-méthyltriphénylméthyl ou 4,4'-diméthyltriphénylméthyl ou 4,4',4''-triméthyltriphénylméthyl.

2. Un composé selon la demande de brevet 1 et de formule I, dans laquelle n, R₂ et R₃ représentent les sujets nommés dans la demande de brevet 1 et R₁ signifie le groupe 9-fluorèneméthoxycarbonyl.

3. Un composé selon la demande de brevet 1 et de formule I, dans laquelle n, R₂ et R₃ représentent les sujets nommés dans la demande de brevet 1 et R₁ signifie le groupe carbobenzoxy.

4. Un composé selon la demande de brevet 1 et de formule I, dans laquelle n, R₂ et R₃ représentent les sujets nommés dans la demande de brevet 1 et R₁ représente hydrogène.

5. Un composé de formule V: 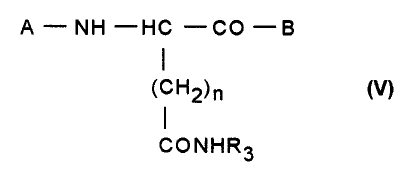 dans laquelle n et R₃ représentent les sujets nommés dans la demande de brevet 1 et les lettres A et B représentent les résidus d'un peptide ou d'un fragment de peptide liés facultativement à un polymère et reliés par leur fonction carboxyle ou amino terminale, respectivement.
